(19) Europäisches Patentamt
European Patent Office
Office européen des brevets

(11) Veröffentlichungsnummer: **0 071 794**
**A1**

(12) # EUROPÄISCHE PATENTANMELDUNG

(21) Anmeldenummer: **82106339.3**

(22) Anmeldetag: **15.07.82**

(51) Int. Cl.³: **C 07 D 231/38**, A 01 N 43/56

(30) Priorität: **25.07.81 DE 3129429**

(43) Veröffentlichungstag der Anmeldung: **16.02.83 Patentblatt 83/7**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Eicken, Karl, Dr., Waldstrasse 63, D-6706 Wachenheim (DE)**
Erfinder: **Plath, Peter, Dr., Berner Weg 24, D-6700 Ludwigshafen (DE)**
Erfinder: **Wuerzer, Bruno, Dr., Dipl.-Landwirt, Ruedigerstrasse 13, D-6701 Otterstadt (DE)**

(54) **5-Amino-1-phenyl-pyrazol-4-carbonsäurederivate, Verfahren zu ihrer Herstellung und ihre Verwendung zur Bekämpfung unerwünschten Pflanzenwuchses.**

(57) Die vorliegende Erfindung betrifft 5-Amino-1-phenyl-pyrazol-4-carbonsäurederivate der Formel

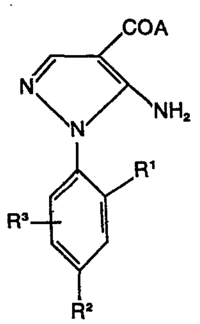

in der
R¹, R², R³ und A die in der Beschreibung genannten Bedeutungen haben, Verfahren zu ihrer Herstellung und ihre Verwendung zur Bekämpfung unerwünschten Pflanzenwuchses.

EP 0 071 794 A1

0071794

BASF Aktiengesellschaft O.Z. 0050/35305

# 5-Amino-1-phenyl-pyrazol-4-carbonsäurederivate, Verfahren zu ihrer Herstellung und ihre Verwendung zur Bekämpfung unerwünschten Pflanzenwuchses

Die vorliegende Erfindung betrifft 5-Amino-1-phenyl-pyrazol-4-carbonsäurederivate und ihre Salze, Verfahren zur Herstellung dieser Verbindungen, Herbizide, welche diese Verbindungen als Wirkstoffe enthalten, sowie Verfahren zur Bekämpfung unerwünschten Pflanzenwuchses mit diesen Verbindungen.

5-Amino-1-phenyl-pyrazol-4-carbonsäureester, wobei der Phenylring chlorsubstituiert ist, sind bekannt (US-PS 3 567 735; Archiv. Pharm. 312 (1979), 703). Sie sind Zwischenprodukte für die Synthese von Diuretika oder antibakteriellen Verbindungen. Über herbizide Eigenschaften dieser Verbindungen ist nichts bekannt.

Es wurde gefunden, daß 5-Amino-1-phenyl-pyrazol-4-carbonsäurederivate der Formel

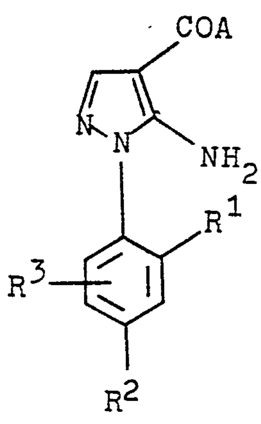

(I),

in der

$R^1$ Methyl, Trifluormethyl, Chlor oder Brom,

$R^2$ Chlor, Brom, Jod oder Methylsulfonyl,

$R^3$ einen Methoxyrest in 5-Stellung, Wasserstoff, Chlor oder Brom bedeuten und

H/HB

A für die Reste $-OR^4$, $-NR^5R^6$ oder $-ON=CR^5R^6$ steht, wobei

$R^4$ einen gegebenenfalls durch Phenyl substituierten Alkenylrest mit 3 bis 12 Kohlenstoffatomen, einen Alkinylrest mit 3 bis 10 Kohlenstoffatomen, einen Phenalkylrest mit 1 bis 8 Kohlenstoffatomen in der Alkylgruppe, einen gegebenenfalls am Phenylring durch Halogen, Alkyl mit 1 bis 4 Kohlenstoffatomen oder Methoxy substituierten Phenoxalkylrest mit 2 bis 8 Kohlenstoffatomen in der Alkylgruppe, einen Cycloalkylmethylrest mit insgesamt 4 bis 7 Kohlenstoffatomen, einen durch eine Alkoxygruppe mit 1 bis 12 Kohlenstoffatomen, eine Alkylthiogruppe mit 1 bis 12 Kohlenstoffatomen, Allyloxy, Propargyloxy, Chlor oder Brom substituierten Alkylrest mit 2 bis 4 Kohlenstoffatomen und

$R^5$ und $R^6$ gleich oder verschieden sind und einen Alkylrest mit 1 bis 6 Kohlenstoffatomen oder zusammen eine Alkylengruppe mit 4 bis 6 Kohlenstoffatomen bedeuten,

oder Salze der Verbindungen der Formel I eine überraschend starke und gleichzeitig selektive herbizide Wirkung besitzen.

5-Amino-1-phenyl-pyrazol-4-carbonsäurederivate der Formel I werden durch Umsetzung eines substituierten Phenylhydrazins der Formel

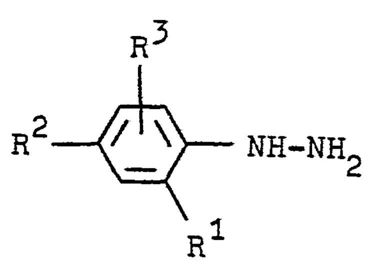

(II),

in der
$R^1$, $R^2$ und $R^3$ die obengenannten Bedeutungen haben, oder eines mineralsauren Salzes des Phenylhydrazins mit einem substituierten 2-Cyanacrylsäurederivat der Formel

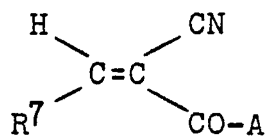
(III),

in der
$R^7$ einen Alkoxyrest mit 1 bis 4 Kohlenstoffatomen, einen Dialkylaminorest mit 1 bis 4 Kohlenstoffatomen in einer Alkylgruppe oder Hydroxy und
A die Reste $-OR^4$ oder $-NR^5R^6$, wobei $R^4$, $R^5$ und $R^6$ die obengenannten Bedeutungen haben, bedeuten,

gegebenenfalls in Gegenwart eines säurebindenden Mittels zu einem substituierten 2'-Phenylhydrazino-2-cyanacrylsäurederivat der Formel

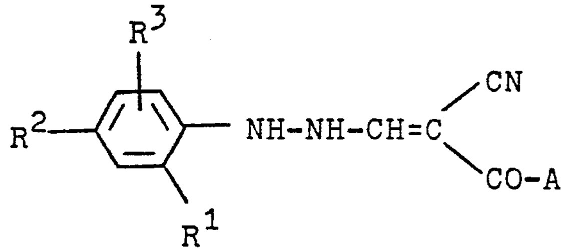
(IV),

in der $R^1$, $R^2$, $R^3$ und A die oben angegebenen Bedeutungen haben, bei einer Temperatur unterhalb 70°C und Cyclisierung der so erhaltenen Verbindung der Formel IV durch Erhitzen auf eine Temperatur oberhalb 70°C (Verfahren A) oder durch Behandeln mit wäßriger Mineralsäure bei einer Temperatur zwischen 0 und 150°C, vorzugsweise zwischen 20 und 100°C, (Verfahren B) erhalten.

Als Lösungsmittel für das Verfahren A eignen sich insbesondere Alkohole, z.B. Methanol, Ethanol; es können jedoch auch Ether, wie Dioxan, Tetrahydrofuran, Anisol, oder Kohlenwasserstoffe, wie Toluol, Xylol, verwendet werden. Nach Beendigung der Umsetzung der Verbindungen der Formeln II und III bzw. nach der Cyclisierung wird die jeweilige Lösung gekühlt, und das gebildete Produkt wird durch Abfiltrieren isoliert und gegebenenfalls durch Umkristallisieren gereinigt. Wird die Cyclisierung nach Verfahren B mit wäßriger Mineralsäure, vorzugsweise 5 bis 38 %iger (Gew.%) Salzsäure oder 5 bis 50 %iger Schwefelsäure, vorgenommen, wird das Reaktionsgemisch nach Ende der Umsetzung mit dem doppelten bis zwanzigfachen Volumen Wasser verdünnt, das Cyclisierungsprodukt wird abgesaugt und unter Zugabe von verdünntem Alkali oder Ammoniak neutral gewaschen und gegebenenfalls umkristallisiert.

Ferner erhält man 5-Amino-1-phenyl-pyrazol-4-carbonsäurederivate der Formel I durch eine einstufige Umsetzung eines substituierten Phenylhydrazins der Formel II mit einem substituierten 2-Cyanacrylsäurederivat der Formel III bei einer Temperatur oberhalb von 70°C (Verfahren C). Als Lösungsmittel eignen sich die für das Verfahren A genannten Solventien, vorzugsweise Alkohole, deren Siedepunkte über 70°C liegen. Die Isolierung der Endprodukte erfolgt in der im Verfahren A beschriebenen Weise. Die 2-Cyanacrylsäurederivate der Formel III werden in mindestens molarer Menge, bezogen auf die substituierten Phenylhydrazine der Formel II, eingesetzt, vorzugsweise in stöchiometrischer Menge. Werden im Verfahren A oder C anstelle der freien Phenylhydrazine der Formel II deren mineralsaure Salze, z.B. Hydrochloride oder Sulfate, eingesetzt, so ist es zweckmäßig, zuerst das substituierte Phenylhydrazin der Formel II durch

einer äquivalenten Menge an Alkalialkoholaten oder Natriumacetat freizusetzen und dann die Umsetzung auszuführen.

Man erhält außerdem 5-Amino-1-phenyl-pyrazol-4-carbonsäurederivate der Formel I, in der A für die Reste $-OR^4$ oder $-O-N=CR^5R^6$ steht, wobei $R^4$, $R^5$ und $R^6$ die oben angegebenen Bedeutungen haben, durch Umsetzung von 5-Amino-1-phenyl-pyrazol-4-carbonsäurealkylestern der Formel

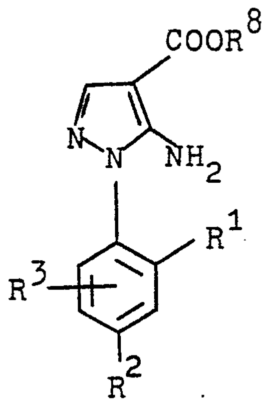

(V),

in der $R^1$, $R^2$ und $R^3$ die obengenannten Bedeutungen haben und $R^8$ Alkyl mit 1 bis 3 Kohlenstoffatomen bedeutet, mit Alkoholen der Formel $R^4OH$ oder Oximen der Formel $HO-N=CR^5R^6$, wobei $R^4$, $R^5$ und $R^6$ die obengenannten Bedeutungen haben, in Gegenwart eines Alkoholates als Katalysator.

Die Reaktionstemperatur liegt dabei oberhalb der Siedetemperatur der Alkoholkomponente des eingesetzten Esters, d.h. im Bereich von 80 bis 250°C. Der Alkohol der Formel $R^4OH$ bzw. das Oxim der Formel $HO-N=CR^5R^6$ werden vorteilhafterweise im Überschuß von 10 bis 1000 Mol je Mol Ausgangsverbindung der Formel V eingesetzt. Als Katalysatoren kommen Alkalialkoholate, insbesondere Alkalimethylate, in Betracht und zwar in Mengen von 1 bis 20 Molprozent, bezogen auf eingesetzten Ester der Formel V. Zur Isolierung des Endprodukts wird der Überschuß des Alkohols der

Formel $R^4OH$ bzw. des Oxims der Formel $HO-N=CR^5R^6$ durch Verdampfen oder, falls wasserlöslich, durch Verdünnen mit Wasser entfernt. Die 5-Amino-1-phenyl-pyrazol-4-carbonsäurederivate der Formel I können, falls erforderlich, durch Fällen als mineralsaure Salze isoliert oder durch Umkristallisieren weiter gereinigt werden.

Als Säuren, die mit den 5-Amino-1-phenyl-pyrazol-4-carbonsäurederivaten der Formel I Salze bilden, kommen starke anorganische Säuren, insbesondere Mineralsäuren oder starke organische Säuren in Betracht, wie Trifluoressigsäure, Schwefelsäure, Chlorwasserstoffsäure oder Bromwasserstoffsäure, Benzolsulfonsäure.

Die Phenylhydrazine der Formel II sind bekannt oder können nach bekannten Methoden hergestellt werden (Houben-Weyl, Methoden der organischen Chemie, Bd. 10/2, S. 180 ff, Georg-Thieme-Verlag, Stuttgart, 1967). Die 2-Cyanacrylsäureester der Formel III sind teilweise bekannt oder können nach bekannten Methoden hergestellt werden (DE-OS 26 35 841; Chem.Ber. (1964) 97, 3397).

$R^4$ im Rest $-OR^4$ für A in Formel I steht beispielsweise für einen gegebenenfalls durch Phenyl substituierten, unverzweigten oder verzweigten Alkenylrest mit 3 bis 12 Kohlenstoffatomen, vorzugsweise mit 3 bis 6 Kohlenstoffatomen, wie Allyl, Crotonyl, Methallyl, 3-Methyl-but-2-enyl, 3-Methyl-but-3-enyl, Hex-5-enyl, Undec-10-enyl, 2-Phenyl-prop-2-enyl, 3-Phenyl-prop-2-enyl, für einen unverzweigten oder verzweigten Alkinylrest mit 3 bis 10 Kohlenstoffatomen, insbesondere mit 3 bis 6 Kohlenstoffatomen, wie Propargyl, But-2-inyl, Hex-5-inyl, für einen Phenalkylrest mit 1 bis 8 Kohlenstoffatomen, vorzugsweise 1 bis 3 Kohlenstoffatomen, in der Alkylgruppe, wie Benzyl, 2-Phenethyl, 2-Phenyl-n-propyl, 3-Phenyl-n-propyl, für einen gegebenenfalls am Phenylring durch Halogen, Alkyl mit 1 bis 4 Kohlenstoffatomen oder Methoxy substituierten Phenoxyalkylrest mit 2 bis 8 Kohlenstoffatomen, vorzugsweise 2 bis 4 Kohlenstoffatomen, in der Alkylgrupppe, wie 2-Phenoxy--ethyl, 3-Phenoxy-n-propyl, 2-Phenoxy-n-propyl, 4-Phenoxy--n-butyl, für einen Cycloalkylmethylrest mit insgesamt 4 bis 7 Kohlenstoffatomen, wie Cyclopropylmethyl, Cyclopentylmethyl, Cyclohexylmethyl, oder für einen durch eine Alkoxygruppe mit 1 bis 12 Kohlenstoffatomen, vorzugsweise mit 1 bis 6 Kohlenstoffatomen, eine Alkylthiogruppe mit 1 bis 12 Kohlenstoffatomen, vorzugsweise 1 bis 6 Kohlenstoffatomen, durch Allyloxy, Propargyloxy, Chlor oder Brom substituierten Alkylrest mit 2 bis 4 Kohlenstoffatomen, wie 2-Methoxy-ethyl, 2-Ethoxy-ethyl, 2-(n-Propoxy)-ethyl, 2-(n--Butoxy)-ethyl, 2-(n-Hexoxy)-ethyl, 3-Methoxy-n-propyl, 2-Methoxy-n-propyl, 2-Chlorethyl, 2-Bromethyl, 3-Chlor-n--propyl, 4-Chlor-n-butyl, 2-Methylthio-ethyl, 2-(n-Propylthio)-ethyl, 2-(n-Butylthio)-ethyl, 4-Methoxy-n-butyl, 3-Methoxy-n-butyl.

$R^5$ und $R^6$ in den Resten $-NR^5R^6$ und $-O-C=NR^5R^6$ für A in Formel I stehen beispielsweise für einen Alkylrest mit 1 bis 6 Kohlenstoffatomen, vorzugsweise 1 bis 4 Kohlenstoffatomen, oder bilden zusammen eine Alkylengruppe mit 4 bis 6 Kohlenstoffatomen. Als Aminoreste der Formel $-NR^5R^6$ kommen somit Dimethylamino, Diethylamino, Di-n-propylamino, Di-n-butylamino, Methylethylamino, Pyrrolidino oder Piperidino in Betracht. Den Oximethern der Formel I liegen beispielsweise folgende Oxime zugrunde: Acetonoxim, Methylethylketonoxim, Diethylketonoxim, Cyclopentenonoxim, Cyclohexanonoxim.

Bevorzugte Verbindungen sind solche mit einem Rest $-OR^4$ für A, wobei $R^4$ einen unverzweigten oder verzweigten Alkenylrest mit 3 bis 12 Kohlenstoffatomen oder einen durch

eine Alkoxygruppe mit 1 bis 12, vorzugsweise 1 bis 6 Kohlenstoffatomen substituierten Alkylrest mit 2 bis 4 Kohlenstoffatomen bedeutet.

Die folgenden Beispiele erläutern die Herstellung der Zwischenprodukte der Formeln IV, V und der Endprodukte der Formel I. In den Beispielen verhalten sich Gewichtsteile zu Volumenteilen wie Kilogramm zu Liter.

Beispiel A

148,1 Gewichtsteile 2,4,6-Trichlorphenylhydrazin werden in eine Lösung von 108,5 Gewichtsteilen Ethoxymethylen-2-cyanessigsäuremethylester in 1000 Volumenteilen Methanol gegeben. Aus der Lösung fällt ein Kristallbrei aus, der nach dreistündigem Rühren, nach Absaugen des Niederschlages und Trocknen unter vermindertem Druck bei 40°C 187,4 Gewichtsteile 2'-(2,4,6-Trichlorphenyl)-hydrazino-2-cyanacrylsäuremethylester vom Fp. 174 bis 175°C liefert.

$C_{11}H_8Cl_3N_3O_2$ (M 320,5)

| | | | |
|---|---|---|---|
| ber.: | C 41,22 | H 2,52 | N 13,11 |
| gef.: | C 40,9 | H 2,8 | N 12,8 |

Beispiel B

Eine Suspension von 21,4 Gewichtsteilen 2,4-Dichlorphenylhydrazinhydrochlorid in 150 Volumenteilen Methanol wird durch Zusatz von ca. 18 Gewichtsteilen 30 %iger Natriumethylatlösung neutralisiert und nach Zugabe von 15,5 Gewichtsteilen Ethoxymethylen-2-cyanessigsäuremethylester 3 Stunden lang bei 25°C gerührt und danach 15 Minuten unter Rückfluß erhitzt. Aus dem Filtrat isoliert man nach dem Verdampfen des Methanols unter vermindertem Druck und Umkristallisieren aus Ethanol (bei 50°C) 17,5 Gewichtsteile 2'-(2,4-Dichlorphenyl)-hydrazino-2-cyanacrylsäuremethylester vom Fp. 154 bis 156°C.

$C_{11}H_9Cl_2N_3O_2$ (M 286)
ber.: C 46,18 H 3,17 N 14,69
gef.: C 46,0 H 3,2 N 14,8

In entsprechender Weise können folgende 2'-Phenylhydrazino-2-cyanacrylsäureester der Formel

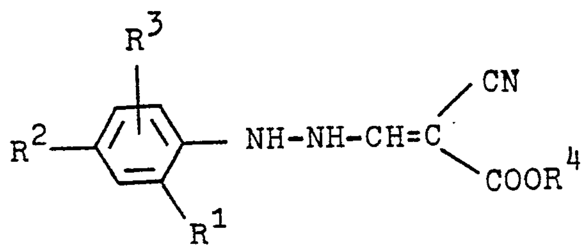

(IV)

hergestellt werden:

| $R^1$ | $R^2$ | $R^3$ | $R^4$ | Fp [°C] |
|---|---|---|---|---|
| Cl | Cl | H | $C_2H_5$ | 175 |
| Cl | Cl | 6-Cl | $C_2H_5$ | 166 |
| Cl | Cl | 6-Cl | i-$C_3H_7$ | 130 |
| $CH_3$ | Cl | H | $CH_3$ | 140 |
| Br | Br | 6-Br | $CH_3$ | 182 |
| Cl | Br | 5-Cl | $CH_3$ | |
| Cl | Cl | 5-Cl | $CH_3$ | 195 |
| $CH_3$ | Br | H | $CH_3$ | |
| $CH_3$ | Br | 6-Br | $CH_3$ | |
| $CH_3$ | Cl | 6-Cl | $CH_3$ | |
| Cl | Cl | 6-Br | $CH_3$ | |
| Cl | Br | 6-Br | $CH_3$ | |
| Cl | Br | 6-Cl | $CH_3$ | |
| Br | Cl | 6-Br | $CH_3$ | |
| Br | Br | H | $CH_3$ | |
| Cl | Cl | 5-$CH_3O$ | $CH_3$ | |

Beispiel C

90,0 Gewichtsteile 2'-(2,4,6-Trichlorphenyl)-hydrazino-2-cyanacrylsäuremethylester werden in 300 Volumenteilen 18 %iger Salzsäure 5 Stunden lang bei 80°C gerührt. Nach dem Abkühlen und Verdünnen mit 500 Volumenteilen Wasser isoliert man nach dem Absaugen und Neutralwaschen mit Wasser und Natriumhydrogencarbonatlösung 70,6 Gewichtsteile 5-Amino-1-(2,4,6-trichlorphenyl)-pyrazol-4-carbonsäuremethylester vom Fp. 179 bis 180°C.

$C_{11}H_8Cl_3N_3O_2$ (M 320,5)

| | | | |
|---|---|---|---|
| ber.: | C 41,22 | H 2,52 | N 13,11 |
| gef.: | C 41,4 | H 2,8 | N 12,6 |

Beispiel D

15,0 Gewichtsteile 2'-(2,4,6-Dichlorphenyl)-hydrazino-2-cyanacrylsäuremethylester werden in 50 Volumenteilen konzentrierter Salzsäure 12 Stunden lang bei 25°C gerührt und danach in 500 Volumenteile Eiswasser eingerührt. Der ausgefallene Niederschlag ergibt nach Absaugen und Neutralwaschen mit Wasser und Natriumhydrogencarbonatlösung 14,2 Gewichtsteile 5-Amino-1-(2,4-dichlorphenyl)-pyrazol-4-carbonsäuremethylester vom Fp. 143 bis 145°C.

$C_{11}H_9Cl_2N_3O_2$ (M 286)

| | | | |
|---|---|---|---|
| ber.: | C 46,18 | H 3,17 | N 14,69 |
| gef.: | C 46,2 | H 3,2 | N 14,7 |

Beispiel E

45,0 Gewichtsteile 2'-(2,4-Dichlorphenyl)-hydrazino-2-cyanacrylsäuremethylester werden in 200 Volumenteilen n-Propanol 6 Stunden lang unter Rückfluß erhitzt. Nach

dem Abkühlen isoliert man durch Absaugen 34,6 Gewichtsteile 5-Amino-1-(2,4-dichlorphenyl)-pyrazol-4-carbonsäuremethylester vom Fp. 144 bis 145°C.

Beispiel F

21,2 Gewichtsteile 2,4,6-Trichlorphenylhydrazin und 15,5 Gewichtsteile Ethoxymethylen-2-cyanessigsäuremethylester werden in 120 Volumenteilen n-Butanol 2 Stunden lang unter Rückfluß erhitzt. Nach dem Abkühlen isoliert man durch Absaugen 22,8 Gewichtsteile 5-Amino-1-(2,4,6-trichlorphenyl)-pyrazol-4-carbonsäuremethylester vom Fp. 180 bis 181°C.

Entsprechend erhält man die folgenden 5-Amino-1-phenyl-pyrazol-4-carbonsäureester der Formel V

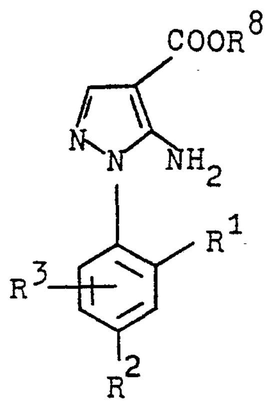

(V),

| $R^1$ | $R^2$ | $R^3$ | $R^8$ | Fp [°C] |
|---|---|---|---|---|
| Cl | Cl | H | $C_2H_5$ | 109 |
| Cl | Cl | 6-Cl | $C_2H_5$ | 105 |
| Cl | Cl | 6-Cl | i-$C_3H_7$ | 152 |
| $CH_3$ | Cl | H | $CH_3$ | 109 |
| Br | Br | 6-Br | $CH_3$ | 194 |
| Cl | Br | 5-Cl | $CH_3$ | 173 |
| Cl | Cl | 5-Cl | $CH_3$ | 175 |
| $CH_3$ | Br | H | $CH_3$ | 123 |

| $R^1$ | $R^2$ | $R^3$ | $R^8$ | Fp [°C] |
|---|---|---|---|---|
| $CH_3$ | Br | 6-Br | $CH_3$ | 175 |
| $CH_3$ | Cl | 6-Cl | $CH_3$ | |
| Cl | Cl | 6-Br | $CH_3$ | 199 |
| Cl | Br | 6-Br | $CH_3$ | 208 |
| Cl | Br | 6-Cl | $CH_3$ | 181 |
| Br | Cl | 6-Br | $CH_3$ | 172 |
| Br | Br | H | $CH_3$ | |
| Cl | Cl | 5-$CH_3O$ | $CH_3$ | 182 |
| Cl | Br | 6-Cl | $CH_3$ | |
| Cl | J | H | $CH_3$ | 180 |
| Cl | H | 3-Cl | $CH_3$ | 164 |
| Cl | Cl | 6-Cl | n-$C_3H_7$ | 92 |
| $CF_3$ | Cl | H | $CH_3$ | 146 |
| Cl | $SO_2CH_3$ | H | $CH_3$ | 215 |

Beispiel 1

20,5 Gewichtsteile 2,6-Dichlor-4-bromphenylhydrazin und 14,5 Gewichtsteile Ethoxymethylen-2-cyanessigsäureallylester werden in 100 Volumenteilen n-Butanol 4 Stunden lang unter Rückfluß erhitzt. Nach dem Verdampfen des Butanols wird der ölige Rückstand in 150 Volumenteilen Ether gelöst, wonach unter Eiskühlung trockenes Chlorwasserstoffgas eingeleitet wird. Aus dem ausgefallenen Hydrochlorid werden nach Verrühren mit gesättigter Natriumhydrogencarbonatlösung, Absaugen und Trocknen unter vermindertem Druck 24,8 Gewichtsteile 5-Amino-1-(2,6-dichlor-4-bromphenyl)-pyrazol-4-carbonsäureallylester vom Fp. 85°C erhalten (Wirkstoff Nr. 6).

$C_{13}H_{10}N_3O_2Cl_2Br$ (M 391)

| | | | |
|---|---|---|---|
| ber.: | C 39,8 | H 2,55 | N 10,77 |
| gef.: | C 39,2 | H 2,7 | N 10,4 |

Beispiel 2

12,7 Gewichtsteile 2,4,6-Trichlorphenylhyrazin und 10,8 Gewichtsteile p-Dimethylamino-α-cyanacrylsäure-N,N--dimethylamid werden in 100 Volumenteilen n-Butanol 12 Stunden lang unter Rückfluß gekocht. Nach dem Verdampfen des Butanols isoliert man durch Anteigen des kristallinen Rückstandes mit Ether 9,4 Gewichtsteile 5-Amino-1-(2,4,6-trichlorphenyl)-pyrazol-4-carbonsäure--N,N-dimethylamid vom Fp. 226 bis 228°C (Wirkstoff Nr. 16).

$C_{12}H_{11}N_4Cl_3O$ (M 333,5)

| | | | |
|---|---|---|---|
| ber.: | C 43,20 | H 3,32 | N 16,79 |
| gef.: | C 43,6 | H 3,2 | N 16,8 |

Beispiel 3

12,0 Gewichtsteile 5-Amino-1-(2,4,6-trichlorphenyl)-pyrazol-4-carbonsäuremethylester, 24,0 Gewichtsteile 3-Methyl--but-2-en-ol und 0,5 Gewichtsteile Natriummethylat werden unter Durchleiten von trockenem Stickstoff 2 Stunden lang unter Rückfluß erhitzt (vollständiger Umsatz). Der überschüssige Alkohol wird unter vermindertem Druck bei 0,5 bis 1,0 mbar/50°C entfernt, der Rückstand wird in 100 ml Ether gelöst und mit trockenem Chlorwasserstoffgas gefällt. Nach dem Absaugen des Hydrochlorids, dem Verrühren mit gesättigter Natriumhydrogencarbonatlösung, Absaugen und Trocknen unter vermindertem Druck bei 50°C erhält man 10,6 g 5-Amino-1-(2,4,6-trichlorphenyl)-pyrazol-4-carbonsäure-3-methyl-but-2-en-yl-ester vom Fp. 102 bis 105°C (Wirkstoff Nr. 10).

$C_{15}H_{14}N_3O_2Cl_2$ (M 374,5)

| | | | |
|---|---|---|---|
| ber.: | C 48,09 | H 3,77 | N 11,22 |
| gef.: | C 48,3 | H 4,0 | N 11,1 |

Beispiel 4

10,0 Gewichtsteile 5-Amino-1-(2,4,6-trichlorphenyl)-pyrazol-4-carbonsäuremethylester, 22,5 Gewichtsteile Acetonoxim und 0,5 Gewichtsteile Natriummethylat werden unter Überleiten von trockenem Stickstoff 8 Stunden lang bei 100°C gerührt (50 % Umsatz). Nach dem Abkühlen wird das Rohprodukt zwischen 150 Volumenteilen Ether und 100 Volumenteilen Wasser verteilt, die organische Phase wird dreimal mit je 200 Volumenteilen Wasser gewaschen und nach dem Trocknen unter vermindertem Druck eingeengt. Aus dem Rückstand erhält man nach Chromatographie an Kieselgel (80 g) mit Essigester-Toluol-Gemisch (3:7) 2,2 Gewichtsteile 5-Amino-1-(2,4,6-trichlorphenyl)-pyrazol-4-carbonsäure-acetonoximester (Wirkstoff Nr. 20) vom Fp. 194 bis 196°C (aus Toluol).

$C_{13}H_{11}N_4O_2Cl_3$ (M 362)

| | | | |
|---|---|---|---|
| ber.: | C 43,18 | H 3,07 | N 15,49 |
| gef.: | C 43,3 | H 3,1 | N 15,2 |

Nach einem der oben beschriebenen Verfahren wurden folgende 5-Amino-1-phenyl-pyrazol-4-carbonsäurederivate der Formel I bzw. ihre Salze hergestellt:

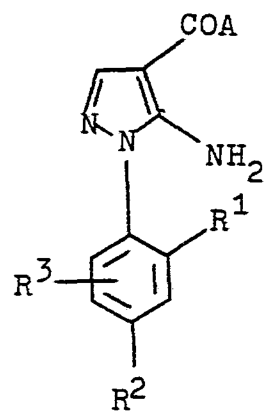

| Nr. | $R^1$ | $R^2$ | $R^3$ | A | Fp [°C] |
|---|---|---|---|---|---|
| 1 | $CH_3$ | Cl | H | $OCH_2CH=CH_2$ | Öl |
| 2 | Cl | Cl | H | $OCH_2CH=CH_2$ | 88- 90 |
| 3 | Cl | Cl | 6-Cl | $OCH_2CH=CH_2$ | 118-120 |
| 4 | Cl | $SO_2CH_3$ | H | $OCH_2CH=CH_2$ | 153 |
| 5 | Cl | Cl | 6-Br | $OCH_2CH=CH_2$ | 135 |
| 6 | Cl | Br | 6-Cl | $OCH_2CH=CH_2$ | 85 |
| 7 | Br | Br | 6-Br | $OCH_2CH=CH_2$ | 148 |
| 8 | Cl | Cl | 6-Cl | $OCH_2-C_6H_5$ | 157 |
| 9 | Cl | Cl | 6-Cl | $OCH_2C(CH_3)=CH_2$ | 110 |
| 10 | Cl | Cl | 6-Cl | $OCH_2CH=C(CH_3)_2$ | 102-105 |
| 11 | Cl | Cl | 6-Cl | $O(CH_2)_2OCH_3$ | 140-142 |
| 12 | Cl | Cl | 6-Cl | $O(CH_2)_4CH=CH_2$ | Öl |
| 13 | Cl | Cl | 6-Cl | $O(CH_2)_9CH=CH_2$ | Öl |
| 14 | Cl | Cl | 6-Cl | $O(CH_2)_2C_6H_5$ | 142-144 |
| 15 | Cl | Cl | 6-Cl | $OCH_2CH=CH_2.HCl$ | 158-160 |
| 16 | Cl | Cl | 6-Cl | $N(CH_3)_2$ | 226-228 |
| 17 | Cl | Cl | 6-Cl | $O(CH_2)_3C_6H_5$ | 112 |
| 18 | Cl | Cl | 6-Cl | $OCH_2CH(CH_3)C_6H_5$ | 60- 65 |
| 19 | Cl | Cl | 6-Cl | $O(CH_2)_2OC_6H_5$ | 164-166 |
| 20 | Cl | Cl | 6-Cl | $ON=C(CH_3)_2$ | 194-196 |

Die Verbindungen der Formel I können in die üblichen Formulierungen übergeführt werden, wie Lösungen, Pulver, Suspensionen oder Dispersionen, Emulsionen, Öldispersionen, Pasten, Stäubemittel, Streumittel, Granulate. Die Anwendungsformen richten sich ganz nach den Verwendungszwecken; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische cyclische

und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlrobenzol, Isophoron usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, äthoxyliertes Isooctylphenol-, Octylphenol-, Nonylphenol, Alkylphenolpolyglykoläther. Tributylphenylpolyglykolether.

Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcelluslose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumringen-, Holz- und Nußschalenmehl, Cellulosepulver und andere feste Trägerstoffe.

Die herbiziden Mittel enthalten im allgemeinen zwischen 0,1 und 95 Gew.%, vorzugsweise zwischen 0,5 und 90 Gew.%, Wirkstoff.

Die Aufwandmengen liegen je nach Zusammensetzung und Wachstumsstadien der Unkrautflora bei 0,1 bis 15 kg und mehr, vorzugsweise zwischen 0,1 und 4 kg Wirkstoff pro Hektar, wobei für eine totale Pflanzenvernichtung die höheren Aufwandmengen zu verwenden sind.

Die Anwendung kann im Vorauflaufverfahren oder vorzugsweise bei Nachauflaufanwendung erfolgen.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Sind Kulturpflanzen bei Blattbehandlung gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by).

Beispiele für Zubereitungen sind:

I. Man vermischt 90 Gewichtsteile des Wirkstoffs 17 mit 10 Gewichtsteilen N-Methyl-alpha-pyrrolidon und erhält eine Lösung, die zur Anwendung in Form kleinster Tropfen geeignet ist.

II. 10 Gewichtsteile des Wirkstoffs 12 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Rizinusöl besteht.

III. 20 Gewichtsteile des Wirkstoffs 8 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Rizinusöl besteht.

IV. 20 Gewichtsteile des Wirkstoffs 13 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht.

V. 80 Gewichtsteile des Wirkstoffs 20 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile des Wirkstoffs 19 werden mit 95 Gewichtsteielen feinteiligem Kaolin innig vermischt. Man erhält auf diese Weise ein Stäubemittel, das 5 Gewichtsprozent des Wirkstoffs enthält.

VII. 30 Gewichtsprozent des Wirkstoffs 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile des Wirkstoffs 2 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

IX. 20 Teile des Wirkstoffs 4 werden mit 12 Teilen Calcium-salz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die herbizide Wirkung der 5-Amino-1-phenyl-pyrazol-4-carbonsäureester der Formel I auf das Wachstum von erwünschten und unerwünschten Pflanzen wird anhand von Gewächshausversuchen gezeigt.

Als Kulturgefäße dienen Plastikblumentöpfe mit 300 $cm^3$ Inhalt, gefüllt mit lehmigem Sand mit etwa 1,5 % Humus als Substrat. Bei Klettenlabkraut wird reichlich Torf (peat) zugesetzt, um ein besseres Auflaufen und Wachstum zu gewährleisten. Die Samen der Testpflanzen werden nach Arten getrennt flach eingesät. Zum Zwecke der Nachauflaufbehandlung zieht man die Testpflanzen je nach Wuchsform erst bis zu einer Höhe von 3 bis 10 cm und behandelt sie danach. Zur Nachauflaufbehandlung werden entweder direkt gesäte und in den gleichen Gefäßen aufgewachsene Pflanzen ausgewählt, oder die Pflanzen werden erst als Keimpflanzen getrennt angezogen und einige

Tage vor der Behandlung in die Versuchsgefäße verpflanzt. Die Aufwandmengen variieren je nach Wirkstoff.

Die Plastikblumentöpfe werden im Gewächshaus aufgestellt, wobei für wärmeliebende Arten wärmere Bereiche (20 bis 35°C) und für solche gemäßigter Klimate 15 bis 25°C bevorzugt werden. Die Versuchsperiode erstreckt sich über 2 bis 3 Wochen. Während dieser Zeit wird das Wachstum der Pflanzen beurteilt. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Versuchen werden die folgenden Pflanzen getestet:

Abutilon theophrasti (Chinesischer Hanf), Amaranthus spp. (zurückgekrümmter Fuchsschwanz), Avena sativa (Hafer), Chenopodium album (Weißer Gänsefuß), Galeopsis spp. (Hohlzahnarten), Galium aparine (Klettenlabkraut), Hordeum vulgare (Gerste), Ipomoea spp. (Prunkwindenarten), Malva neglecta (Wegmalve), Solanum nigrum (Schwarzer Nachtschatten), Triticum aestivum (Weizen), Veronica persica (Persischer Ehrenpreis), Viola tricolor (Stiefmütterchen).

Bei diesen Versuchen zeigen die Verbindungen Nr. 3, 12, 13 und 17 bei Nachauflaufbehandlung eine gute Wirkung gegen eine Reihe breitblättriger unerwünschter Pflanzen. Getreidearten wie Gerste und Weizen werden weitgehend geschont oder nur vorübergehend geringfügig geschädigt. Die Verbindung Nr. 8 zeigt im Nachauflaufverfahren eine Wirkung gegen das breitblättrige Unkraut Galeopsis spp., Amaranthus spp. und Solanum nigrum.

In Anbetracht der Vielseitigkeit der Applikationsmethode können die erfindungsgemäßen Herbizide noch in einer weiteren großen Zahl von Kulturpflanzen zur Beseitigung unerwünschten Pflanzenwuchses eingesetzt werden.

Im einzelnen seien folgende Nutzpflanzen genannt:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojabohne |

| Botanischer Name | Deutscher Name |
|---|---|
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakatuschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolben-hirse |
| Petroselinum crispum spp. tuberosum | Wurzelpetersilie |
| Picea abies | Rotfichte |
| Abies alba | Weißtanne |
| Pinus spp. | Kiefer |

| Botanischer Name | Deutscher Name |
|---|---|
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis | Kuhbohne |
| Vitis vinifera | Weinrebe |
| Zea mays | Mais |

Zur Verbreiterung des Wirkungsspektrums und zur Erzielung synergistischer Effekte können die neuen 5-Amino-1-phenyl-pyrazol-4-carbonsäureester mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Patentansprüche

1. 5-Amino-1-phenyl-pyrazol-4-carbonsäurederivate der Formel

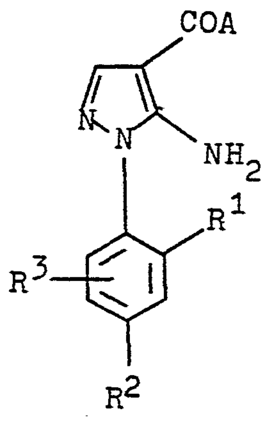

(I),

in der

$R^1$ Methyl, Trifluormethyl, Chlor oder Brom,

$R^2$ Chlor, Brom, Jod oder Methylsulfonyl,

$R^3$ einen Methoxyrest in 5-Stellung, Wasserstoff, Chlor oder Brom bedeuten und

A für die Reste $-OR^4$, $-NR^5R^6$ oder $-ON=CR^5R^6$ steht, wobei

$R^4$ einen gegebenenfalls durch Phenyl substituierten Alkenylrest mit 3 bis 12 Kohlenstoffatomen, einen Alkinylrest mit 3 bis 10 Kohlenstoffatomen, einen Phenalkylrest mit 1 bis 8 Kohlenstoffatomen in der Alkylgruppe, einen gegebenenfalls am Phenylring durch Halogen, Alkyl mit 1 bis 4 Kohlenstoffatomen oder Methoxy substituierten Phenoxalkylrest mit 2 bis 8 Kohlenstoffatomen in der Alkylgruppe, einen Cycloalkylmethylrest mit insgesamt 4 bis 7 Kohlenstoffatomen, einen durch eine Alkoxygruppe mit 1 bis 12 Kohlenstoffatomen, eine Alkylthiogruppe mit 1 bis 12 Kohlenstoffatomen, Allyloxy, Propargyloxy, Chlor oder Brom substituierten Alkylrest mit 2 bis 4 Kohlenstoffatomen und

$R^5$ und $R^6$ gleich oder verschieden sind und einen Alkylrest mit 1 bis 6 Kohlenstoffatomen oder zusammen eine Alkylengruppe mit 4 bis 6 Kohlenstoffatomen bedeuten,

oder Salze der Verbindungen der Formel I.

2. 5-Amino-1-phenyl-pyrazol-4-carbonsäurederivate der Formel I gemäß Anspruch 1, dadurch gekennzeichnet, daß A für einen Rest $-OR^4$ steht, wobei $R^4$ einen Alkenylrest mit 3 bis 12 Kohlenstoffatomen oder einen durch eine Alkoxygruppe mit 1 bis 12 Kohlenstoffatomen substituierten Alkylrest mit 2 bis 4 Kohlenstoffatomen bedeutet.

3. Verfahren zur Herstellung von 5-Amino-1-phenyl-pyrazo-4-carbonsäurederivaten der Formel I gemäß Anspruch 1, dadurch gekennzeichnet, daß man ein substituiertes Phenylhydrazin der Formel

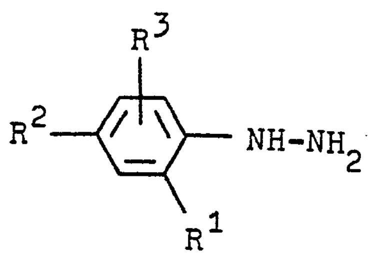

(II),

in der
$R^1$, $R^2$ und $R^3$ die im Anspruch 1 genannten Bedeutungen haben, oder mineralsaure Salze des Phenylhydrazins mit einem 2-Cyanacrylsäurederivat der Formel

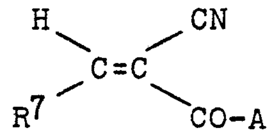

(III),

in der

$R^7$ einen Alkoxyrest mit 1 bis 4 Kohlenstoffatomen, einen Dialkylaminorest mit 1 bis 4 Kohlenstoffatomen in einer Alkylgruppe oder Hydroxy und

A die Reste $-OR^4$ oder $-NR^5R^6$, wobei $R^4$, $R^5$ und $R^6$ die im Anspruch 1 genannten Bedeutungen haben,

bedeuten,

gegebenenfalls in Gegenwart eines säurebindenden Mittels zu einem substituierten 2'-Phenylhydrazino-2-cyanacrylsäurederivat der Formel

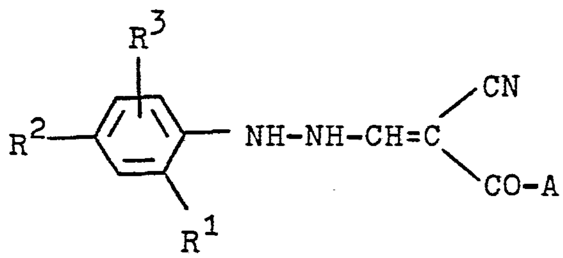 (IV),

in der $R^1$, $R^2$, $R^3$ und A die oben angegebenen Bedeutungen haben, bei einer Temperatur unterhalb 70°C umsetzt und die so erhaltene Verbindung durch Erhitzen auf eine Temperatur oberhalb 70°C in einem organischen Lösungsmittel oder durch Behandeln mit wäßriger Mineralsäure bei einer Temperatur zwischen 0 und 150°C in an sich üblicher Weise cyclisiert.

4. Verfahren zur Herstellung von 5-Amino-1-phenyl-pyrazol-4-carbonsäurederivaten gemäß Anspruch 3, dadurch gekennzeichnet, daß man die Umsetzung von Phenylhydrazin der Formel II mit 2-Cyanacrylsäurederivat der Formel III in einer Stufe bei einer Temperatur oberhalb von 70°C in einem organischen Lösungsmittel durchführt.

5. Verfahren zur Herstellung von 5-Amino-1-phenyl-pyrazol-4-carbonsäurederivaten der Formel I gemäß Anspruch 1, in der A für die Reste $-OR^4$ oder $-O-N=CR^5R^6$ steht, wobei $R^4$, $R^5$ und $R^6$ die im Anspruch 1 genannten Bedeutungen haben, <u>dadurch gekennzeichnet</u>, daß man einen 5-Amino-1-phenyl-4-carbonsäurealkylester der Formel

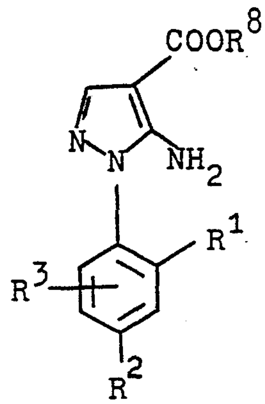

(V),

in der $R^1$, $R^2$ und $R^3$ die im Anspruch 1 genannten Bedeutungen haben und $R^8$ Alkyl mit 1 bis 3 Kohlenstoffatomen bedeutet, mit einem Alkohol der Formel $R^4OH$ oder einem Oxim der Formel $HO-N=CR^5R^6$, wobei $R^4$, $R^5$ und $R^6$ die im Anspruch 1 genannten Bedeutungen haben, in Gegenwart eines Alkoholates als Katalysator umsetzt.

6. Herbizid, enthaltend ein 5-Amino-1-phenyl-pyrazol-4-carbonsäurederivat der Formel

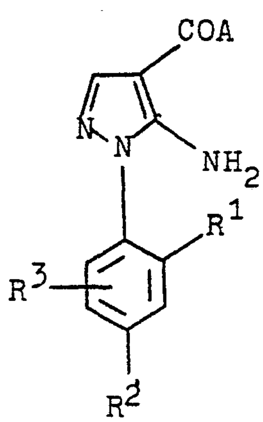

(I),

in der

$R^1$ Methyl, Trifluormethyl, Chlor oder Brom,

$R^2$ Chlor, Brom, Jod oder Methylsulfonyl,

$R^3$ einen Methoxyrest in 5-Stellung, Wasserstoff, Chlor oder Brom bedeuten und

A für die Reste $-OR^4$, $-NR^5R^6$ oder $-ON=CR^5R^6$ steht, wobei

$R^4$ einen gegebenenfalls durch Phenyl substituierten Alkenylrest mit 3 bis 12 Kohlenstoffatomen, einen Alkinylrest mit 3 bis 10 Kohlenstoffatomen, einen Phenalkylrest mit 1 bis 8 Kohlenstoffatomen in der Alkylgruppe, einen gegebenenfalls am Phenylring durch Halogen, Alkyl mit 1 bis 4 Kohlenstoffatomen oder Methoxy substituierten Phenoxalkylrest mit 2 bis 8 Kohlenstoffatomen in der Alkylgruppe, einen Cycloalkylmethylrest mit insgesamt 4 bis 7 Kohlenstoffatomen, einen durch eine Alkoxygruppe mit 1 bis 12 Kohlenstoffatomen, eine Alkylthiogruppe mit 1 bis 12 Kohlenstoffatomen, Allyloxy, Propargyloxy, Chlor oder Brom substituierten Alkylrest mit 2 bis 4 Kohlenstoffatomen und

$R^5$ und $R^6$ gleich oder verschieden sind und einen Alkylrest mit 1 bis 6 Kohlenstoffatomen oder zusammen eine Alkylengruppe mit 4 bis 6 Kohlenstoffatomen bedeuten,

oder Salze einer solchen Verbindung.

7. Herbizid, enthaltend inerte Zusatzstoffe und ein 5-Amino-1-phenyl-pyrazol-4-carbonsäurederivat der Formel

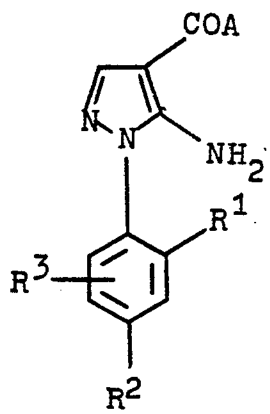

in der

$R^1$ Methyl, Trifluormethyl, Chlor oder Brom,

$R^2$ Chlor, Brom, Jod oder Methylsulfonyl,

$R^3$ einen Methoxyrest in 5-Stellung, Wasserstoff, Chlor oder Brom bedeuten und

A für die Reste $-OR^4$, $-NR^5R^6$ oder $-ON=CR^5R^6$ steht, wobei

$R^4$ einen gegebenenfalls durch Phenyl substituierten Alkenylrest mit 3 bis 12 Kohlenstoffatomen, einen Alkinylrest mit 3 bis 10 Kohlenstoffatomen, einen Phenalkylrest mit 1 bis 8 Kohlenstoffatomen in der Alkylgruppe, einen gegebenenfalls am Phenylring durch Halogen, Alkyl mit 1 bis 4 Kohlenstoffatomen oder Methoxy substituierten Phenoxalkylrest mit 2 bis 8 Kohlenstoffatomen in der Alkylgruppe, einen Cycloalkylmethylrest mit insgesamt 4 bis 7 Kohlenstoffatomen, einen durch eine Alkoxygruppe mit 1 bis 12 Kohlenstoffatomen, eine Alkylthiogruppe mit 1 bis 12 Kohlenstoffatomen, Allyloxy, Propargyloxy, Chlor oder Brom substituierten Alkylrest mit 2 bis 4 Kohlenstoffatomen und

$R^5$ und $R^6$ gleich oder verschieden sind und einen Alkylrest mit 1 bis 6 Kohlenstoffatomen oder zusammen eine Alkylengruppe mit 4 bis 6 Kohlenstoffatomen bedeuten,

oder Salze einer solchen Verbindung.

8. Herbizid nach Anspruch 7, dadurch gekennzeichnet, daß es ein 5-Amino-1-phenyl-pyrazol-4-carbonsäurederivat der Formel I mit einem Rest $-OR^4$ für A enthält, wobei $R^4$ einen Alkenylrest mit 3 bis 12 Kohlenstoffatomen oder einen durch eine Alkoxygruppe mit 1 bis 12 Kohlenstoffatomen substituierten Alkylrest mit 2 bis 4 Kohlenstoffatomen bedeutet.

9. Verfahren zur Bekämpfung unerwünschten Pflanzenwuchses, dadurch gekennzeichnet, daß man die Pflanzen oder ihren Standort mit einer herbizid wirksamen Menge eines 5-Amino-1-phenyl-pyrazol-4-carbonsäurederivates der Formel I gemäß Anspruch 1 oder eines Salzes eines solchen 5-Amino-1-phenyl-pyrazol-4-carbonsäurederivats behandelt.

Europäisches Patentamt

# EUROPÄISCHER RECHERCHENBERICHT

0071794

Nummer der Anmeldung

EP 82 10 6339

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| | --- | | C 07 D 231/38<br>A 01 N 43/56 |
| A | EP-A-0 026 034 (FISONS)<br>*Patentansprüche*<br>--- | 1,3,4,6,7,8,9 | |
| A,P | EP-A-0 034 945 (MAY & BAKER)<br>*Patentansprüche*<br>----- | 1,3,4,6,7,8,9 | |
| | | | RECHERCHIERTE SACHGEBIETE (Int. Cl. 3) |
| | | | C 07 D 231/00 |

Der vorliegende Recherchenbericht wurde für alle Patentansprüche erstellt.

| Recherchenort | Abschlußdatum der Recherche | Prüfer |
|---|---|---|
| DEN HAAG | 17-11-1982 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82